(19) Europäisches Patentamt
European Patent Office
Office européen des brevets

(11) **EP 1 924 633 B1**

(12) **EUROPÄISCHE PATENTSCHRIFT**

(45) Veröffentlichungstag und Bekanntmachung des Hinweises auf die Patenterteilung:
**21.01.2015 Patentblatt 2015/04**

(21) Anmeldenummer: **06792970.3**

(22) Anmeldetag: **23.08.2006**

(51) Int Cl.:
*A61K 8/81* (2006.01)          *A61K 47/20* (2006.01)
*A61K 47/32* (2006.01)          *C08F 2/24* (2006.01)
*C08L 31/04* (2006.01)          *C08L 33/02* (2006.01)

(86) Internationale Anmeldenummer:
**PCT/EP2006/065590**

(87) Internationale Veröffentlichungsnummer:
**WO 2007/025922 (08.03.2007 Gazette 2007/10)**

(54) **WÄSSRIGE POLYVINYLACETATDISPERSIONEN MIT HOHER SCHERSTABILITÄT**

AQUEOUS POLYVINYL ACETATE DISPERSIONS HAVING HIGH SHEARING STABILITY

DISPERSIONS AQUEUSES D'ACETATE DE POLYVINYLE A HAUTE STABILITE AU CISAILLEMENT

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **02.09.2005 DE 102005042039**

(43) Veröffentlichungstag der Anmeldung:
**28.05.2008 Patentblatt 2008/22**

(73) Patentinhaber: **BASF SE**
**67056 Ludwigshafen (DE)**

(72) Erfinder:
• **ANGEL, Maximilian**
  **67105 Schifferstadt (DE)**
• **KOLTER, Karl**
  **67117 Limburgerhof (DE)**

(56) Entgegenhaltungen:
**EP-A1- 0 019 716      EP-A1- 1 069 169**
**EP-A2- 1 142 934      EP-A2- 1 571 164**
**FR-A- 2 428 058        US-A- 5 863 975**
**US-A- 6 069 217        US-A1- 2005 039 635**

**Beschreibung**

Die vorliegende Erfindung betrifft Polyvinylacetatdispersionen mit verbesserter Scherstabilität sowie ein Verfahren zur Herstellung solcher Dispersionen.

Polyvinylacetatdispersionen und deren Verwendung für eine Vielzahl von Anwendungen sind an sich bekannt.

Aus der US 5 252 704 sind in Wasser redispergierbare Polymerpulver bekannt, die unter Verwendung von Polyvinylpyrrolidon (PVP) als Dispersionsmittel hergestellt werden. Zur Herstellung der Polymerpulver werden unter anderem auch Vinylester in üblicher Emulsionspolymerisation eingesetzt. Vor der Sprühtrocknung wird der Emulsion PVP zugesetzt. Die Polymerpulver sind vor allem als Additive für Zementmischungen vorgesehen.

In der DE 197 09 532 A ist die Verwendung von redispergierbaren Polymerpulvern oder Polymergranulaten zum Überziehen von pharmazeutischen oder agrochemischen Darreichungsformen beschrieben, wobei die Pulver bzw. Granulate aus 10 bis 95 Gew.-% Polyvinylacetat und aus 5 bis 90 Gew.-% eines N-Vinylpyrrolidon-haltigen Polymers sowie gegebenenfalls weiteren Zusatzstoffen bestehen.

Aus der deutschen Patentanmeldung P 102004011349.1 ist die Herstellung von redispergierbaren Polymerisaten, insbesondere von (Meth)acrylaten, in Gegenwart von ionischen Emulgatoren beschrieben. Ganz allgemein werden auch Polyvinylester erwähnt.

Für die Herstellung von pharmazeutischen Darreichungsformen werden wie in DE 197 09 532 häufig Polymerpulver eingesetzt, die zur Herstellung dieser Darreichungsformen in Wasser redispergiert werden müssen. Die Gründe für diese Herstellung von redispergierbaren Pulvern liegen darin, dass es häufig nicht möglich ist, die wässrigen Zubereitungen entsprechend mikrobiologisch zu stabilisieren, so dass sie die hohen Anforderungen, die an Einsatzstoffe für Arzneimittel gestellt werden, erfüllen. So dürfen beispielsweise kein mikrobiologischer Befall und auch keine Teilchenvergröberung oder gar Koagulation bzw. Sedimentation auftreten, weil dadurch die sichere Herstellung des Arzneimittels gefährdet wird. Die wässrigen Zubereitungen sind oft nur über Wochen stabil. Um zu einer längeren Haltbarkeit zu kommen, werden die wässrigen Zubereitungen in Pulver überführt, aus denen wiederum vor der Anwendung durch Einrühren in Wasser eine wässrige Zubereitung herstellt werden muss.

Daher sind insbesondere für pharmazeutische und kosmetische Anwendungen erhöhte Anforderungen an die Scherstabilität solcher Dispersionen zu stellen, damit die Bildung von Koagulat verhindert wird.

In der WO 02/26845 ist die Herstellung von Polyvinylacetatdispersionen durch radikalische Polymerisation von Vinylacetat in Gegenwart von Schutzkolloiden und ionischen Emulgatoren beschrieben. Allerdings lassen die so erhaltenen Dispersionen hinsichtlich der Scherstabilität noch Raum für Verbesserungen.

Die Tatsache, dass Stabilisatoren in pharmazeutischen Dispersionen die Filmbildung negativ beeinflussen, ist lange bekannt. So wird in "Aqueous Polymeric coatings for Pharmaceutical Dosage Forms", 2nd Edition, Ed. James W. McGinity 1997, Marcel Dekker Inc., New York, Seiten 56-67, ausgeführt, dass Surfactants und wassserlösliche Additive sehr nachteilige Wirkungen auf die Verfilmung und die Filmeigenschaften haben. Insbesondere wird Natriumlaurylsulfat erwähnt, das die Struktur, die mechanischen und Permeationseigenschaften von Filmen verändert, weil es während der Filmbildung in kleine Zwischenräume zwischen den Polymerteilchen wandert.. Auch hydrophile Verbindungen wie Polyethoxylate können sehr nachteilige Auswirkungen auf die Filme haben. So kommt es beispielsweise zu Phasenseparation, Flocculation, Beschleunigung der Wirkstofffreisetzung, Verschlechterung der mechanischen Eigenschaften, Kristallisation der Emulgatoren und ähnlichen unerwünschten Phänomenen.

Aus der GB 1450599 ist die Herstellung eines salzstabilen Polyvinylacetats bekannt wobei ein Acrylsäureanteil von 3 Gew.-% im Monomerengemisch eingesetzt wird.

Aufgabe der vorliegenden Erfindung war es, wässrige Polyvinylacetatdispersionen mit verbesserter Scherstabilität bei möglichst geringem Emulgatorgehalt zu finden.

Demgemäß wurden wässrige Polyvinylacetatdispersionen gefunden, welche erhältlich sind durch radikalische Polymerisation von Vinylacetat in Gegenwart von 0,1 bis 0,6 Gew.-% Acrylsäure oder Methacrylsäure oder Mischungen davon, mit der Maßgabe, dass das Gesamtgewicht an Monomeren 100 Gew.-% beträgt, und 0,001 bis 1 Gew.-%, bezogen auf das Gesamtgewicht der Monomeren, mindestens eines ionischen Emulgators.

Bevorzugt wird Acrylsäure eingesetzt.

Als in der erfindungsgemäßen Dispersion enthaltener ionischer Emulgator kann ein üblicher anionischer Emulgator vorgesehen werden, wie z.B. Alkalimetall- und Ammoniumsalze von Alkylsulfaten (Alkylrest: $C_8$ bis $C_{16}$), von Alkylsulfonsäuren (Alkylrest: $C_8$ bis $C_{16}$), von Schwefelsäurehalbestern ethoxylierter Alkanole (EO-Grad: 4 bis 100, Alkylrest: $C_{12}$ bis $C_{16}$), und ethoxylierter Alkylphenole (EO-Grad 3 bis 50, Alkylrest: $C_4$ bis $C_{12}$), und von Alkylarylsulfonsäuren (Alkylrest; $C_9$ bis $C_{18}$). Als weitere anionische Emulgatoren haben sich ferner Verbindungen wie Dowfax 2A1 (Marke der Dow Chemical Company) als vorteilhaft erwiesen. Bevorzugt ist Natriumlaurylsulfat. Der ionische Emulgator wird in Konzentrationen von 0,001 bis 1 Gew.-%, vorzugsweise 0,1 bis 0,5 Gew.-%, bezogen auf den Gesamtmonomergehalt, eingesetzt.

Zusätzlich können gewünschtenfalls noch übliche nicht-ionische Emulgatoren eingesetzt werden. Bevorzugt wird jedoch nur ein anionischer Emulgator verwendet.

Gemäß einer bevorzugten Ausführungsform enthalten die Dispersionen ein Schutzkolloid. Das Schutzkolloid ist bevorzugt in einer Menge zwischen 1 und 20 Gew.-%, besonders bevorzugt 5 bis 12 Gew.-%, bezogen auf das Gesamtgewicht an Monomeren, enthalten. Das in der erfindungsgemäßen Dispersion enthaltene Schutzkolloid ist bevorzugt Polyvinylpyrrolidon, das besonders bevorzugt einen K-Wert von 20 bis 40 aufweist. Es können zusätzlich noch weitere wasserlösliche oder wasserquellbare Schutzkolloide eingesetzt werden, wie z.B. Cellulosederivate, bevorzugt Hydroxypropylmethylcellulose, Methylcellulose oder Hydroxyethylcellulose, Galactomannan, Pectin, Xanthan, Polyvinylalkohol, Acrylat-Methacrylat Copolymere, Natriumcarboxymethylstärke, Cellulose, abgebaute Stärken, Maltodextrine etc. Die Schutzkolloide können dabei vor, während und nach der Polymerisation zugegeben werden. Bevorzugt ist die Zugabe zur Polymerisation.

Die vorliegende Erfindung betrifft auch ein Verfahren zur Herstellung einer wässrigen Polymerdispersion, bei dem Vinylacetat durch radikalische Polymerisation in Gegenwart von mindestens einem ionischen Emulgator polymerisiert wird, welches dadurch gekennzeichnet ist, dass die Polymerisation in Gegenwart von 0,1 bis 0,6 Gew-% Acrylsäure oder Methacrylsäure, mit der Maßgabe, dass das Gesamtgewicht an Monomeren 100 Gew.-% beträgt, und 0,001 bis 1 Gew.-%, bezogen auf die Gesamtmenge an Monomeren, des ionischen Emulgators.

Bevorzugt erfolgt die Polymerisation als Emulsionspolymerisation in Wasser in Gegenwart eines Schutzkolloids. Dabei beträgt das Gew.-Verhältnis zwischen Schutzkolloid und ionischem Emulgator mindestens 4 : 1 und bis zu 100:1. Das Gew.-Verhältnis zwischen Monomeren und Schutzkolloid liegt zwischen 99:1 und 4:1, bevorzugt zwischen 19 : 1 und 4 : 1.

Der für die Polymerisation eingesetzte radikalische Initiator ist bevorzugt Na-, K- oder Ammoniumperoxodisulfat, aber auch andere an sich übliche radikalische Initiatoren wie Wasserstoffperoxid, organische Peroxide, Hydroperoxide oder Azoverbindungen sind - auch in Verbindung mit Redoxkomponenten wie z.B. Ascorbinsäure - möglich.

Weiterhin kann es sich empfehlen, die Polymerisation in Gegenwart eines Puffersystems durchzuführen. Das erfindungsgemäß eingesetzte Puffersystem ist bevorzugt ein basisch wirkendes Reagenz, wie z.B. Salze einer einer Säure, ausgewählt aus der Gruppe, bestehend aus Kohlensäure, Borsäure, Essigsäure, Zitronensäure und Phosphorsäure, handelt.

Bei der erfindungsgemäßen wässrigen Polymerdispersion handelt es sich vorzugsweise um eine sogenannte "geregelte" Polymerdispersion, d.h. die Polymerisation wird in Gegenwart eines Polymerisationsreglers ausgeführt, wobei sich als Regler besonders schwefelhaltige Verbindungen wie z.B. Thioglykol, t-Dodecylmercaptan, n-Dodecylmercaptan und Ethylhexylthioglykolat eignen, die u.a. dazu führen, dass sich die erfindungsgemäß bevorzugten K-Werte von 45 bis 200, besonders bevorzugt 60 bis 100, insbesondere 65 bis 85, gemessen an einer 0,1 gew.-%igen Lösung in Tetrahydrofuran, einstellen lassen und dass die resultierenden Polymere schwefelhaltige Endgruppen aufweisen. Die Gesamtmenge des Reglers, üblicherweise zwischen 0,05 und 1 %, bevorzugt zwischen 0,1 und 0,5 %, jeweils bezogen auf den Gesamtmonomergehalt, wird bevorzugt in den Emulsionszulauf eingebracht.

Die Emulsionspolymerisation wird in an sich bekannter Weise bei Temperaturen von 40°C bis 95°C, bevorzugt bei Temperaturen unter 80◦C durchgeführt.

Bevorzugt wird dieses Verfahren nach einem halbkontinuierlichen Zulaufverfahren ausgeführt, wobei die Gesamtmenge des Schutzkolloids in der Vorlage vorgelegt wird. Eine weitere bevorzugte Verfahrensweise besteht darin, dass der ionische Emulgator zu mehr als 50 von 100 Teilen in der Vorlage enthalten ist.

Die nach dem erfindungsgemäßen Verfahren erhaltenen Dispersionen weisen üblicherweise einen Feststoffgehalt von 10 bis 45 Gew.-%, bevorzugt von 15 bis 35 Gew.-% auf.

Die erfindungsgemäßen Dispersionen zeichnen sich durch eine hohe Stabilität bei vergleichsweise niedrigem Emulgatorgehalt aus. Das erfindungsgemäße Verfahren bietet den Vorzug einer verbesserten Reproduzierbarkeit hinsichtlich der Eigenschaften der erhaltenen Dispersionen. Auch die weiteren anwendungstechnischen Eigenschaften sind im Vergleich zu Homopolyvinylacetatdispersionen äußerst zufriedenstellend.

Für die Herstellung von Überzügen ist - neben dem Molekulargewicht respektive dem K-Wert - die Teilchengröße der Dispersionsteilchen von besonderer Bedeutung. Die erfindungsgemäße Polymerdispersion weist daher bevorzugt Dispersionsteilchen auf, die eine mittlere Teilchengröße von nur 50 bis 300 nm, vorzugsweise von 100 bis 200 nm aufweisen. Die Bestimmung erfolgt in der üblichen Weise z.B. mittels Ultrazentrifuge, Photonenkorrelationsspektroskopie oder durch Bestimmung der Lichtdurchlässigkeit. Die Partikelgröße wird üblicherweise über die Emulgatorkonzentration gesteuert. Die erfindungsgemäß erhaltenen Dispersionsteilchen sind sehr feinteilig, obschon nur eine sehr geringe Menge eines Emulgators bei der Polymerisation verwendet wird.

Gegenstand der Erfindung sind außerdem pharmazeutische und kosmetische Darreichungsformen, enthaltend das erfindungsgemäße Polymerisat als Überzugsmittel und/oder filmbildenden Hilfsstoff, wobei das Polymerisat, das sowohl als wässrige Dispersion als auch als Polymerpulver eingesetzt werden kann, erhältlich ist durch das eingangs beschriebene Verfahren.

Bei den pharmazeutischen Darreichungsformen handelt es sich bevorzugt u.a. um Filmtabletten, Filmmikrotabletten, Dragees, überzogene Pastillen, Kapseln, Kristalle, Granulate oder Pellets.

Als pharmazeutische Wirkstoffe können erfindungsgemäß neben Arzneistoffen auch Vitamine, Nahrungser-

gänzungsmittel oder dietätische Wirkstoffe eingesetzt werden.

Die Darreichungsformen weisen eine verzögerte Freisetzung des Wirkstoffs auf. Solche Formen werden auch als Formen mit retardierter Freisetzung bezeichnet. Retardiert bedeutet in diesem Zusammenhang, dass die Freisetzung über einen Zeitraum von 4 bis 36 Stunden erfolgt und dass die Freisetzung von 80 % des Wirkstoffs frühestens nach vier Stunden erfolgt ist. Damit unterscheiden sich Retardformen von schnellfreisetzenden Formen, bei denen 80 % des Wirkstoffs spätestens nach einer Stunde freigesetzt sind.

Die Formen setzen ph-unabhängig frei. Eine pH-unabhängige Freisetzung bedeutete, dass die Freisetzung in künstlichem Magensaft (0.1 n HCl; pH 1,2) sich von der Freisetzung in künstlichem Darmsaft (Phosphatpuffer; pH 6,8) nicht unterscheidet. D.h. die Differenz in der Freisetzung zu jedem Prüfzeitpunkt soll nicht mehr als 10% betragen.

Zur Anwendung für pharmazeutische Darreichungsformen können den Dispersionen zusätzlich übliche pharmazeutisch akzeptable Hilfsstoffe ausgewählt aus der Gruppe bestehend aus Antischaummitteln, Füllstoffen, Farbstoffen, Pigmenten, Antioxidantien, Konservierungsmitteln, Glanzverstärkern und Weichmachern in den hierfür üblichen Mengen zugegeben werden. Bei niedrig löslichen Wirkstoffen kann es sich empfehlen zur Steuerung der Freisetzung zusätzlich einen Porenbildner zuzusetzen. Als Porenbildner eignen sich beispielsweise Hydroxypropylmethylcellulose, Polyvinylalkohol-Polyethylenglykol-Pfropfcopolymere wie Kollicoat® IR, Polyvinylalkohole, Vinylpyrro lidon-Homo- und Copolymere, natürliche Polysaccharide, oder niedermolekulare wasserlösliche Stoffe wie Zucker, Zuckeralkohole, anorganische Salze. Solche Porenbildner können je nach Löslichkeit in Mengen von 1 bis 30, bevorzugt 2 bis 20 Gew.-%, bezogen auf Filmbildner.

Insgesamt kann der Anteil an Hilfsstoffen bis zu 50 Gew.-%, bezogen auf den Feststoffgehalt der Dispersion, betragen.

Ferner können auch wasserunlösliche aber wasserquellbare Stoffe in kleiner Korngröße wie mikrokristalline Cellulose, quervernetzte Natriumcarboxymethylcellulose oder - stärke und quervernetztes Polyvinylpyrrolidon eingesetzt werden.

Ein besonderer Vorteil der wässrigen Dispersionen liegt unter anderem in ihrer geringen Neigung zum Schäumen und damit verminderter Neigung zur Stippenbildung. Außerdem weisen die Dispersionen eine sehr gute Stabilität auf, was sich an der Scherstabilität und Sedimentationsstabilität ablesen lässt. Die erfindungsgemäß erhaltenen Überzüge weisen neben dem niedrigen Emulgatorgehalt weitere Vorzüge auf. So ist die Klebrigkeit der Filme gegenüber vergleichbaren kommerziellen Mitteln verringert. Zudem weisen die Überzüge hervorragende mechanische Eigenschaften auf. Aufgrund des reduzierten Emulgatoranteils und der verbesserten Filmbildung ergeben sich eine stärkere Retardierung sowie geringere Veränderungen der Wirkstofffreisetzungen bei Lagerung. Curingeffekte, d.h. Veränderungen der Wirkstofffreisetzung bei erhöhter Temperatur sind ebenfalls minimal ausgeprägt bzw. gar nicht vorhanden.

Die erfindungsgemäßen Dispersionen können auch als Gemisch mit anderen Dispersionen eingesetzt werden. So lassen sie sich zum Beispiel in Kombination mit Acrylatdispersionen einsetzen, beispielsweise mit den kommerziell erhältlichen Kollicoat®- oder Eudragit®-Typen. Hierzu gehören z.B. Polyacrylate Dispersion 30 Per Cent Ph. Eur. (Kollicoat® EMM 30 D, Eudragit® NE 30 D), Ammonio Methacrylate Copolymer Type A and B USP/NF (Eudragit® RL und RS) und Methacrylic Acid Copolymer Type C (Kollicoat® MAE 30 DP, Eudragit® L 30-55). Insbesondere eignet sich auch die Kombinationen mit Acrylatdispersionen wie sie in der deutschen Patentanmeldung P 102004011349.1 beschrieben sind. Die erfindungsgemäßen Dispersionen lassen sich auch sehr gut mit Ethylcellulosedispersionen wie z.B. Aquacoat® ECD oder Surelease® kombinieren, wobei insbesondere die Flexibillität der an sich spröden Ethylcellulose verbessert wird.


Herstellung der Polymerisate


Allgemeine Vorschrift


Das Polymerisationsgefäß wurde mit Stickstoff gespült. Das VE-Wasser (vollentsalztes Wasser), das Natriumlaurylsulfat (als 15 gew.-%ige wässrige Lösung) sowie Kollidon 30 und anschließend die Teilmenge Zulauf 02 wurden unter Rühren (120 Upm) in den Polymerisationskessel gegeben (Vorlage). Die Vorlage wurde auf 75°C Innentemperatur (IT) geheizt. Bei 65°C IT wurde der Zulauf 01 (7 gew.-%ige wässrige Natriumperoxodisulfatlösung) schnell zugegeben. Bei Erreichen von 75°C wurde mit den Zuläufen 02 (Emulsionszulauf) und 03 (7 gew.-%ige wässrige Natriumperoxodisulfatlösung) begonnen. Die Zuläufe 02 und 03 wurden über einen Zeitraum von 2 bzw. 3 Stunden zugegeben. Nach Ende der Zuläufe 02 und 03 wurde zwei Stunden nachpolymerisiert. Dann wurde auf 85 °C aufgeheizt und 2 h Vakuumdestilliert (bei ca. 200 mbar).

Das Polymerisationsgemisch wurde auf 20°C gekühlt und mit 1 %iger Natronlauge auf pH 4,5 bis 5 eingestellt.

Kollidon 30 = Polyvinylpyrrolidon mit K-Wert 30 (BASF)

Beispiel 1

Nach der allgemeinen Vorschrift wurde eine Dispersion mit einem Monomerverhältnis VAc / As = 99,5/0,5 Gew.-% hergestellt. Der Emulgatoranteil betrug 0,25 Gew.%, bezogen auf das Monomerengewicht.

Vorlage

| | |
|---|---|
| 559,0 g | VE-Wasser |
| 4,80 g | Natriumlaurylsulfat (15 gew.-%ige wässrige Lösung) |
| 40,00 g | Kollidon 30 |
| 85,6 g | von Zulauf 02 |

Zulauf 01

| | |
|---|---|
| 8,7 g | Natriumperoxodisulfat (7 gew.-%ige wässrige Lösung) |

Zulauf 02

| | |
|---|---|
| 450,3 g | VE-Wasser |
| 0,28 g | Natriumlaurylsulfat (fest, d.h. 100 gew.-%ig) |
| 2,0 g | Natriumacetat x 3 H2O |
| 398,0 g | Vinylacetat |
| 2,0 g | Acrylsäure |
| 0,96 g | t-Dodecylmercaptan |

Zulauf 03

| | |
|---|---|
| 17,6 g | Natriumperoxodisulfat (2,5 gew.-%ige wässrige Lösung) |

Die bläulich weiße wässrige Dispersion wies einen Feststoffgehalt von 29 Gew.% auf. Die mittlere Teilchengröße betrug 160 nm. Der K-Wert, gemessen in einer 0,1 Gew.-%igen Lösung in THF, betrug 75.

Beispiel 2

Es wurde gearbeitet wie im Beispiel 1. Allerdings wurden in der Vorlage 6,35 g (als 15% gew.-%ige Lösung) und im Zulauf 02 0,37 g Natriumlaurylsulfat (fest, d.h. 100 gew.-%ig) verwendet. Anstelle von t-Dodecylmercaptan wurde 2-Ethylhexylthioglykolat verwendet.
Der Emulgatoranteil betrug 0,33 Gew.%, bezogen auf das Monomerengewicht.
Die bläulich weiße wässrige Dispersion wies einen Feststoffgehalt von 29 Gew.% auf. Die mittlere Teilchengröße betrug 150 nm. Der K-Wert, gemessen in einer 0,1 Gew.-%igen Lösung in THF, betrug 73.

Vergleichsbeispiel A

Analog zu der Herstellung gemäß Beispiel 1 wurde die Polymerisation unter Verwendung von 100 Gew.-% Vinylacetat als Monomer durchgeführt. Es entstand keine stabile Dispersion.

Bestimmung der Scherstabilität

Allgemeine Vorschrift

Die zu prüfende Dispersion wird über ein Sieb mit 125 μm-Maschenweite gegeben und 100 g des Filtrats in ein 250-ml-Becherglas eingewogen. Ein Noppenrührer (d = 36 mm mit 4 Noppen von 5 x 3 mm auf dem äußeren und in einem Abstand von 5 mm 4 Noppen von 4 x 3 mm auf dem inneren Kranz, alle 1 cm lang) wird in das Rührwerk eingespannt.

Die Unterkante des Noppenrührers soll 0,5 cm vom Boden des Becherglases entfernt sein. Das Becherglas wird ebenfalls befestigt und die Dispersion wird 15 min. bei 2.000 UPM gerührt und anschließend über ein Sieb von 125 μm Maschenweite abgegossen. Der eventuell anfallende Rückstand wird auf einen, Filter überführt, welcher zuvor bei 105°C eine Stunde getrocknet und anschließend gewogen wurde.

Der Filter mit dem Rückstand wird dann nochmals bei 105°C eine Stunde im Trockenschrank getrocknet.

Nach dem Abkühlen im Exsikkator wird der Filter auf der Analysenwaage zurückgewogen.

Zur Auswertung wird die Menge an Rührkoagulat berechnet:

$$\text{Menge an Rührkoagulat in \%} = \frac{(\text{Filter} + \text{Rückstand} - \text{Filterleergewicht}) \times 100}{\text{Feststoffgehalt der Dispersion in \%}}$$

Je höher die Menge an Rührkoagulat, umso geringer ist die Scherstabilität der geprüften Dispersion.

Vergleichsbeispiel B

Zum Vergleich wurde die Scherstabilität einer reinen Polyvinylacetat-Dispersion mit einem Emulgatorgehalt von 1,004 Gew.-% gemäß Beispiel 1 der WO 02/26845 bestimmt.

| Dispersion gemäß Bsp. Nr. | Rührkoagulat in Gew.-% |
|---|---|
| 1 | 0 |
| 2 | 0 |
| A | 3 |
| B | 0,15 |

**Patentansprüche**

1. Wässrige Polymerdispersion, hergestellt durch radikalische Polymerisation von Vinylacetat in Gegenwart von 0,1 bis 0,6 Gew.-%Gew.-% Acrylsäure oder Methacrylsäure oder Mischungen davon, mit der Maßgabe, dass das Gesamtgewicht an Monomeren 100 Gew.-% beträgt, und 0,001 bis 1 Gew.-% mindestens eines ionischen Emulgators.

2. Wässrige Polymerdispersion nach Anspruch 1, hergestellt unter Verwendung von Acrylsäure.

3. Wässrige Polymerdispersion nach Anspruch 1 oder 2, enthaltend als ionischen Emulgator einen anionischen Emulgator.

4. Wässrige Polymerdispersion nach Anspruch 3, **dadurch gekennzeichnet, dass** es sich bei dem anionischen Emulgator um Natriumlaurylsulfat handelt.

5. Wässrige Polymerdispersion nach einem der Ansprüche 1 bis 4, enthaltend ein Schutzkolloid.

6. Wässrige Polymerdispersion nach einem der Ansprüche 1 bis 5, wobei es sich bei dem Schutzkolloid um Polyvinylpyrrolidon mit einem K-Wert von 20 bis 40 handelt.

**7.** Wässrige Polymerdispersion nach einem der Ansprüche 1 bis 6, wobei der Emulgator in Mengen von 0,1 bis 0,5 Gew.-% eingesetzt wird.

**8.** Wässrige Polymerdispersion nach einem der Ansprüche 1 bis 7, wobei die mittlere Teilchengröße der Dispersionsteilchen von 50 bis 300 nm.

**9.** Wässrige Polymerdispersion nach einem der Ansprüche 1 bis 9, wobei die mittlere Teilchengröße der Dispersionsteilchen von 100 bis 200 nm beträgt.

**10.** Verfahren zur Herstellung einer wässrigen Polymerdispersion gemäß einem der Ansprüche 1 bis 9, durch radikalische Polymerisationvon Vinylacetat in Gegenwart von mindestens einem ionischen Emulgator, , **dadurch gekennzeichnet, dass** die Polymerisation in Gegenwart von 0,1 bis 0,6 Gew.-% Acrylsäure oder Methacrylsäure oder Mischungen davon, mit der Maßgabe, dass das Gesamtgewicht an Monomeren 100 Gew.-% beträgt, und 0,001 bis 1 Gew.-% mindestens eines ionischen Emulgators erfolgt.

**11.** Verfahren nach Anspruch 10, **dadurch gekennzeichnet, dass** die Polymerisation in Gegenwart eines Schutzkolloids erfolgt..

**12.** Pulverförmige Polymerisate, erhalten aus einer wässrigen Dispersion gemäß einem der Ansprüche 1 bis 11.

**13.** Verwendung von wässrigen Dispersionen oder pulverförmigen Polymerisaten gemäß einem der Ansprüche 1 bis 12 in pharmazeutischen oder kosmetischen Darreichungsformen.

**14.** Verwendung nach Anspruch 13 , wobei die Dispersionen als zusätzliche Hilfsmittel pharmazeutisch akzeptable Hilfsstoffe ausgewählt aus der Gruppe bestehend aus Antischaummittteln, Füllstoffen, Farbstoffen, Pigmenten, Anitoxidantien, Konservierungsmitteln, Porenbildnern, Glanzverstärkern und Weichmachern enthalten.

**15.** Pharmazeutische Darreichungsformen mit verzögerter Wirkstofffreisetzung, enthaltend mindestens einen pharmazeutischen Wirkstoff, wobei der Wirkstoff mit einem Filmüberzug, erhalten aus einer wässrigen Dispersion oder einem Polymerisat gemäß einem der Ansprüche 1 bis 11, überzogen ist.

## Claims

**1.** An aqueous polymer dispersion prepared by free-radical polymerization of vinyl acetate in the presence of from 0.1 to 0.6% by weight of acrylic acid or methacrylic acid or mixtures thereof, with the proviso that the total weight of monomers is 100% by weight, and from 0.001 to 1% by weight of at least one ionic emulsifier.

**2.** The aqueous polymer dispersion according to claim 1, prepared by using acrylic acid.

**3.** The aqueous polymer dispersion according to claim 1 or 2, comprising an anionic emulsifier as ionic emulsifier.

**4.** The aqueous polymer dispersion according to claim 3, wherein the anionic emulsifier is sodium lauryl sulfate.

**5.** The aqueous polymer dispersion according to any of claims 1 to 4, comprising a protective colloid.

**6.** The aqueous polymer dispersion according to any of claims 1 to 5, where the protective colloid is polyvinylpyrrolidone having a K value of from 20 to 40.

**7.** The aqueous polymer dispersion according to any of claims 1 to 6, where the emulsifier is employed in amounts of from 0.1 to 0.5% by weight.

**8.** The aqueous polymer dispersion according to any of claims 1 to 7, where the average particle size of the dispersion particles is from 50 to 300 nm.

**9.** The aqueous polymer dispersion according to any of claims 1 to 9, where the average particle size of the dispersion particles is from 100 to 200 nm.

**10.** A process for preparing an aqueous polymer dispersion according to any of claims 1 to 9, by free-radical polymerization of vinyl acetate in the presence of at least one ionic emulsifier, which comprises the polymerization taking place in the presence of from 0.1 to 0.6% by weight of acrylic acid or methacrylic acid or mixtures thereof, with the proviso that the total weight of monomers is 100% by weight, and from 0.001 to 1% by weight of at least one ionic emulsifier.

**11.** The process according to claim 10, wherein the polymerization takes place in the presence of a protective colloid.

**12.** A polymer in powder form obtained from an aqueous dispersion according to any of claims 1 to 11.

**13.** The use of aqueous dispersions or polymers in powder form according to any of claims 1 to 12 in pharmaceutical or cosmetic formulations.

**14.** The use according to claim 13, where the dispersions comprise as additional aids pharmaceutically acceptable excipients selected from the group consisting of antifoams, fillers, colorants, pigments, antioxidants, preservatives, pore formers, gloss improvers and plasticizers.

**15.** A pharmaceutical formulation with delayed release of active ingredient, comprising at least one active pharmaceutical ingredient, where the active ingredient is coated with a film coating obtained from an aqueous dispersion or a polymer according to any of claims 1 to 11.


**Revendications**

**1.** Dispersion aqueuse de polymère, préparée par polymérisation radicalaire d'acétate de vinyle en présence de 0,1 à 0,6% en poids d'acide acrylique ou d'acide méthacrylique ou de leurs mélanges, à condition que le poids total de monomères soit de 100% en poids, et de 0,001 à 1% en poids d'au moins un émulsifiant ionique.

**2.** Dispersion aqueuse de polymère selon la revendication 1, préparée en utilisant de l'acide acrylique.

**3.** Dispersion aqueuse de polymère selon la revendication 1 ou 2, contenant, comme émulsifiant ionique, un émulsifiant anionique.

**4.** Dispersion aqueuse de polymère selon la revendication 3, **caractérisée en ce qu'**il s'agit, pour l'émulsifiant anionique, de laurylsulfate de sodium.

**5.** Dispersion aqueuse de polymère selon l'une quelconque des revendications 1 à 4, contenant un colloïde de protection.

**6.** Dispersion aqueuse de polymère selon l'une quelconque des revendications 1 à 5, où il s'agit, pour le colloïde de protection, de polyvinylpyrrolidone présentant une valeur K de 20 à 40.

**7.** Dispersion aqueuse de polymère selon l'une quelconque des revendications 1 à 6, l'émulsifiant étant utilisé en des quantités de 0,1 à 0,5% en poids.

**8.** Dispersion aqueuse de polymère selon l'une quelconque des revendications 1 à 7, la grosseur moyenne des particules de la dispersion étant de 50 à 300 nm.

**9.** Dispersion aqueuse de polymère selon l'une quelconque des revendications 1 à 9, la grosseur moyenne des particules de la dispersion étant de 100 à 200 nm.

**10.** Procédé pour la préparation d'une dispersion aqueuse de polymère selon l'une quelconque des revendications 1 à 9, par polymérisation radicalaire d'acétate de vinyle en présence d'au moins un émulsifiant ionique, **caractérisé en ce que** la polymérisation a lieu en présence de 0,1 à 0,6% en poids d'acide acrylique ou d'acide méthacrylique ou de leurs mélanges, à condition que le poids total de monomères soit de 100% en poids, et de 0,001 à 1% en poids d'au moins un émulsifiant ionique.

**11.** Procédé selon la revendication 10, **caractérisé en ce que** la polymérisation a lieu en présence d'un colloïde de protection.

12. Polymères sous forme de poudre, obtenus à partir d'une dispersion aqueuse selon l'une quelconque des revendications 1 à 11.

13. Utilisation de dispersions aqueuses ou de polymères sous forme de poudre selon 1"une quelconque des revendications 1 à 12 dans des formes d'administration pharmaceutiques ou cosmétiques.

14. Utilisation selon la revendication 13, les dispersions contenant, comme adjuvants supplémentaires, des matières auxiliaires choisies dans le groupe constitué par les antimousses, les charges, les colorants, les pigments, les antioxydants, les conservateurs, les agents porogènes, les agents de renforcement de la brillance et les plastifiants.

15. Forme d'administration pharmaceutique présentant une libération retardée des substances actives, contenant au moins une substance active pharmaceutique, la substance active étant revêtue d'un revêtement de film obtenu à partir d'une dispersion aqueuse ou d'un polymère selon l'une quelconque des revendications 1 à 11.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5252704 A
- DE 19709532 A
- DE P102004011349
- DE 19709532
- WO 0226845 A
- GB 1450599 A

**In der Beschreibung aufgeführte Nicht-Patentliteratur**

- Aqueous Polymeric coatings for Pharmaceutical Dosage Forms. Marcel Dekker Inc, 1997, 56-67